# EUROPEAN PATENT APPLICATION

(11) **EP 0 596 725 A2**
(43) Date of publication of application: **11.05.1994**
(21) Application number: 93308793.4
(22) Date of filing: 03.11.1993
(51) Int. Cl.: C12P 21/08, G01N 33/53, A61K 39/395

(54) **Drug resistance-conferring protein, antibodies reactive therewith and utilities thereof**

(30) Priority: 05.11.1992 US 971884
(71) Applicant: ELI LILLY AND COMPANY, Indianapolis, Indiana 46285 (US)
(72) Inventor: Starling, James Jacob, Carmel, Indiana 46032 (US)
(74) Representative: Hudson, Christopher Mark

(57) **Abstract**

Monoclonal and genetically engineered antibodies which react with P190, a protein associated with anthracycline resistance are disclosed. The P190 reactive antibodies are useful in determining anthracycline resistance and purification of P190. P190 and antibodies reactive therewith are useful for screening P190 antagonists.

## Description

Anthracyclines represent an important class of oncolytic agents. Doxorubicin, an anthracycline, which is also known in the art as Adriamycin™, is a drug of choice in the clinical management of breast cancer. Therapy with anthracyclines such as doxorubicin is complicated by the appearance of the anthracycline resistant phenotype which limits or negates the oncolytic activity of doxorubicin.

Topoisomerase inhibitors represent a further class of oncolytic agents. Epipodophyllotoxins such as Etoposide® and Teniposide® are topoisomerase inhibitors which are useful in the therapy of neoplasms of the testis, small-cell lung and other lung, breast, Hodgkin's disease, non-Hodgkin's lymphomas, acute granulocytic leukemia and Karposi's sarcoma. The therapeutic utility of the epipodophylotoxins is limited by the appearance of the epipodophyllotoxin resistant phenotype.

The problem of drug resistance is not limited to the anthracyclines or epipodophyllotoxins. Successful treatment of human tumors with various chemotherapeutic agents is limited by the development of drug resistance. *See* Bellamy, W.T., Dalton, W.S., and Dorr, R.T., Cancer Investigation, **8**: 547, (1990); Pastan, I., and Gottesman, M.M., Annu. Rev. Med., **42**: 277, (1991); and Kuzmich, S. and Tew, K.D., Medicinal Res. Rev., **11**: 185, (1991).

One form of multi-drug resistance, mdr, is mediated by a membrane bound 170-180 kD energy-dependent efflux pump designated as P-glycoprotein, P-gp *Id.* P-gp has been shown to play a major role in the intrinsic and acquired resistance of a number of human tumors against hydrophobic, natural product drugs. *Id* Drugs that act as substrates for and are consequently detoxified by P-gp include the vinca alkaloids (vincristine and vinblastine), anthracyclines (Adriamycin), and epipodophyllotoxins (etoposide). While P-gp associated mdr is a major determinant in tumor cell resistance to chemotherapeutic agents, it is clear that the phenomenon of mdr is multifactorial and involves a number of different mechanisms. *See* Kuzmich, S. and Tew, K.D., *supra.* One such alternative pathway for resistance to anthracyclines involves the emergence of a 190 kD protein that is not P-gp. *See* McGrath, T., Latoud, C., Arnold, S.T., Safa, A.R., Felsted, R.S., and Center, M.S. Biochem. Pharmacol., **38**: 3611, (1989). P190 is not found on the plasma membrane but rather appears to be localized predominantly in the endoplasmic reticulum *See* Marquardt, D. and Center, M.S. Cancer Res., **52**: 3157, (1992).

P190 possesses a nucleotide binding domain that is homologous with the ATP binding site of P-gp *See* Marquardt, D., McCrone, S., and Center M.S., Cancer Res., **50**: 1426, (1990). The mechanism(s) utilized by P190 to confer resistance to Adriamycin is not well understood but may involve the intracellular redistribution of Adriamycin away from the nucleus. *See* Marquardt, D. and Center, M.S.,*supra.* Adriamycin is an inhibitor of topoisomerase II (Beck, W.T., Bull. Cancer, **77**: 1131, (1990), which is an enzyme involved in DNA replication. Redistribution of Adriamycin away from the nucleus would therefore be an important component in cellular resistance to this drug. The studies published to date on P190 have utilized cell lines selected *in vitro* for resistance to Adriamycin (McGrath, T., Latoud, C., Arnold, S.T., Safa, A.R., Felsted, R.S., and Center, M.S., *supra;* Marquardt, D. and Center, M.S., *supra;* and Marquardt, D., McCrone, S., and Center M.S. Cancer Res., *supra.* The association of P190 with drug resistance was made by sodium dodecyl sulfate polyacrylamide gel electrophoresis (SDS-PAGE) of radioactive extracts prepared from Adriamycin-resistant HL60/Adr human leukemia cells labeled with 8-azido-alpha[³²P]ATP. *See* McGrath, T., Latoud, C., Arnold, S.T., Safa, A.R., Felsted, R.S., and Center, M.S., *supra.* The drug-resistance phenotype conferred by P190 is not limited to the anthracyclines. Epipodophyllotoxin resistance is linked to P190 expression. The IC₅₀s of HL60/S cells treated with Adriamycin and Etoposide were 0.011 µg/ml and 0.39 µg/ml respectively. The IC₅₀s for HL60/Adr cells (a HL60-derived cell line which is resistant to doxorubicin) treated with Adriamycin and Etoposide were 2.2 µg/ml and >10 µg/ml respectively. HL60/S and HL60/Adr cell lines do not express P-glycoprotein. HL60/Adr expresses P190. Thus, resistance to the anthracyclines and epipodophyllotoxins results from P190 expression.

Monoclonal antibodies (MoAbs) reactive with P190 have not previously been described. Marquardt, D., McCrone, S., and Center M.S., *supra* reported the cross-reactivity of polyclonal antisera prepared against synthetic peptides corresponding to the ATP binding region of mdr with P190 and the inability to generate non cross reactive P190 specific monoclonal anitbodies. The cross reactivity of the polyclonal synthetic peptide specific antibodies of the prior art with mdr compromises the utility of such antisera in determining the molecular basis of drug resistance.

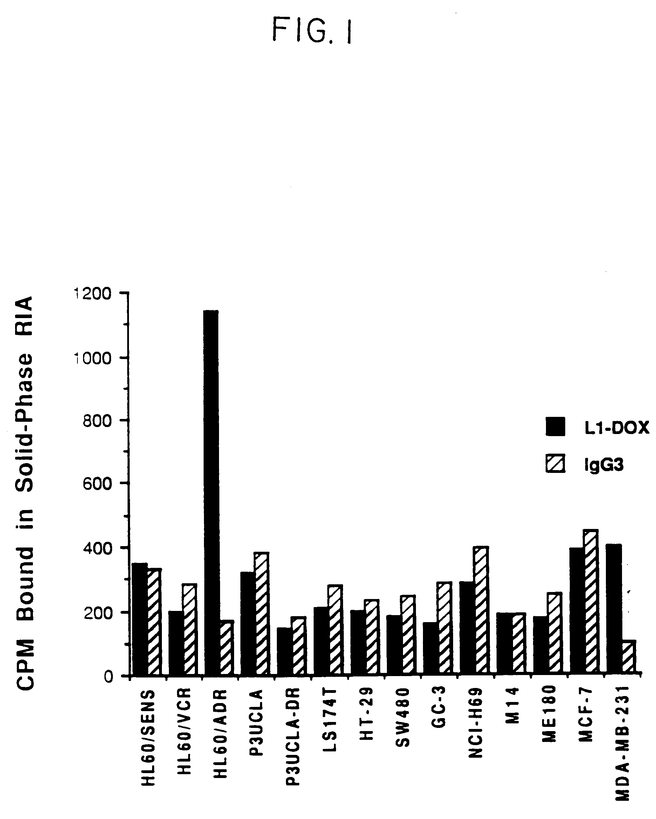

Figure 1. depicts the immunoreactivity of L1-Dox against a panel of human tumor cell lines.

The present invention has surprisingly overcome the problems in the art and provides monoclonal antibodies and genetically engineered equivalents thereof which are reactive with P190, a protein of 190,000 molecular weight, which is associated with the anthracycline and epipodophyllotoxin resistant phenotype and which do not cross react with mdr. The monoclonal antibodies of the present invention are useful for identifying anthracycline resistant cells and thus are useful in the clinical determination of appropriate oncolytic therapy. L1-Dox, a P190 reactive monoclonal antibody is illustrative of the P190 reactive monoclonal antibodies of the present invention. The hybridoma which produces the L1-Dox monoclonal antibody is also designated L1-Dox. The L1-Dox hybridoma was deposited in the American Tissue Culture Collection (ATCC) on November 3, 1992 where it will be publicly available under the accession number HB11174 upon issuance of a patent in a country which is a signatory of the Budapest Treaty.

L1-Dox is useful for preparing P190 in substantial quantities. A variety of immunoadsorbent matrices are known to those of ordinary skill in the art. Cyanogen bromide activated Sepharose® (Pharmacia) is preferred for preparation of L1-Dox immunoadsorbents. Preparation and use of the Cyanogen bromide Sepharose-L1-Dox immunoadsorbent is described in the following Examples.

The illustrative P190 reactive antibody, L1-Dox was prepared by immunizing BALB/c mice with a doxorubicin resistant cell-line. The availability of L1-Dox allows the facile preparation of P190 in substantially pure form. P190 is a preferred immunogen for preparation of P190 reactive monoclonal antibodies. Methods for preparation of monoclonal antibodies are now well known in the art. P190 reactive monoclonal antibodies are prepared according to the art recognized methods which comprises the steps of immunizing an immunocompetent mammal with P190; fusing the lymphocytes of the immunized immunocompetent mammal with appropriate fusion partners; screening the hybridomas for P190 reactivity; and cloning the P190 specific hybridomas by limiting dilution or equivalent techniques. Protocols for monoclonal antibody production are provided in *Selected Methods In Cellular Immunology,* Mishell, B. B. and Shiigi, S. M., Ed (W. H. Freeman and Company, New York, New York, 1980) and *Methods In Enzymology,* Volume 73 Part B, Langone, J. J. and Vunakis, H. V., Ed, (Academic Press, Inc., New York, New York, 1981). Numerous species including rats, mice, rabbits, goats, and humans are useful as a source of immunized lymphocytes for the fusion protocols utilized to generate hybridomas. BALB/c mice are especially preferred as the source of the immune cells for production of P190 specific antibodies. Example 1C of the present disclosure teaches a method for determining whether a hybridoma supernatant reacts with P190. The availability of a family of P190 specific antibodies and the ability to isolate substantial quantities of P190 via immunoaffinity chromatography enable the development of numerous assay systems for detecting agents which bind to P190. One such assay system comprises radiolabeling P190 specific antibodies with ¹²⁵I and measuring displacement of the radiolabeled P190 specific antibody from solid phase P190 which occurs when an agent is present which binds to P190. Numerous other assay systems are also readily adaptable to detect agents which bind P190. The aforementioned assay systems are discussed in Methods in Enzymology, Vol 73, Part B, *supra,* the contents of which are herein incorporated by reference. Skilled artisans are directed to Section II of Methods in Enzymology, Vol 73, Part B, *supra,* which discusses labeling of antibodies and antigens, and Section IV, which discusses immunoassay methods.

Agents which inhibit the function of P190 offer significant clinical opportunities because by reversing the anthracycline/epipodophyllotoxin resistant phenotype they allow resumption of therapy with oncolytics of these classes of oncolytics. L1-Dox binds the P190 antigen thereby inhibiting P190 function. The P190 inhibitors identified with the facile screening methods taught in the present specification will likely supplant L1-Dox as the preferred P190 antagonist due to the large size of L1-Dox, the location of P190, and the immunogenicity of murine antibodies. Genetically engineered antibodies which retain the epitope specificity of monoclonal antibodies are now known in the art and provide a less immunogenic molecule. Such genetically engineered antibodies are contemplated in the present invention. Chimeric antibodies are described in U.S. Patent No. 4,816,567, which issued March 28, 1989. The entire contents of U.S. Patent No. 4,816,567 (Cabilly) are herein incorporated by reference. A further approach to production of genetically engineered antibodies is provided in U.S. Patent No. 4,816,397, which also issued March 28, 1989. The entire contents of U.S. Patent No. 4,816,397 (Boss) are herein incorporated by reference. The approach of U.S. Patent 4,816,397 has been further refined as taught in European Patent Publication No. 0 239 400, which published September 30, 1987. The teachings of European Patent Publication No. 0 239 400 (Winter) are the preferred format for the genetic engineering of the P190 reactive monoclonal antibodies of the invention. The Winter technology involves the replacement of complementarity determining regions CDRs of a human antibody with the CDRs of a murine monoclonal antibody thereby converting the specificity of the human antibody to the specificity of the murine antibody which was the source of the CDR regions. The CDR technology affords a molecule containing minimal murine sequence and thus is less immunogenic. Single chain antibody technology is yet another variety of genetic engineered antibody which is now well known in the art. *See* Bird, R. E., *et al.* 1988 Science **242**:423-426. The single chain antibody technology involves joining the binding regions of heavy and light chains with a polypeptide sequence to generate a single polypeptide having the binding specificity of the antibody from which it was derived. The aforementioned genetic engineering approaches provide the skilled artisan with numerous means to generate molecules which retain the binding characteristics of the parental antibody while affording a less immunogenic format.

A further aspect of the present invention is the utility of the P190 reactive antibodies to provide clinical information regarding whether a given tissue sample contains P190. The ability to determine whether a tumor will be resistant to anthracyclines such as doxorubicin or epipodophyllotoxins such as Etoposide® or Teniposide® merely by evaluating a biopsy sample with a P190 specific monoclonal antibody for the presence of P190 affords the clinician with critical information in that it allows the selection and implementation of alternative chemotherapeutic approaches in cases where the presence of P190 evidences anthracycline/epipodophyllotoxin resistance. The clinical management of cancer requires early detection and efficient therapy and thus the ability to avoid treatment of a drug resistant tumor with an ineffective drug is of vital importance.

The Examples which follow are intended to further illustrate the present invention and are not to be interpreted as limiting on the scope thereof. The Examples provide both experimental detail and results where appropriate.

### Example 1

### Preparation of P190 specific monoclonal antibodies.

### A Cell lines.

HL60 human acute myeloblastic leukemia cells (HL60/s) and HL60 cells isolated for resistance to Adriamycin (HL60/Adr) or vincristine (HL60/Vinc) were obtained from Dr. Melvin Center, Kansas State University, Manhattan, KS. Drug resistant cell-lines are known in the art. *See* McGrath, T., Latoud, C., Arnold, S.T., Safa, A.R., Felsted, R.S., and Center, M.S., *supra* ; Marquardt, D. and Center, M.S., *supra,* and Marquardt, D., McCrone, S., and Center M.S., *supra.* Drug resistant cell-lines are generated by slowly escalating the concentration of the agent towards which the resistant phenotype is being selected. The cells were grown as a suspension culture in RPMI tissue culture medium (GIBCO) supplemented with 10% fetal calf serum and 50 µg/ml gentamicin to a density of 1x10⁴ to 1x10⁵ cells/ml.

### B. Immunizations and Hybridoma Production.

HL60/Adr cells were centrifuged and the cell pellets were washed 3x in phosphate-buffered saline (PBS). The cells were resuspended in PBS and a group of 5 female Balb/c mice (Harlan Sprague-Dawley, Indianapolis, IN) were intraperitoneally (i.p.) injected as follows: day 0, 1.7x10⁷ cells; day 7, 1.7x10⁷ cells; day 14, 2x10⁷ cells. On day 19 blood was removed from the mice and the reactivity of 1:10 and 1:100 dilutions of the plasma fraction was assayed against HL60/Adr cells in a solid-phase radioimmunoassay (RIA). The RIA was performed in substantial accordance with the method reported in *See generally* Starling, J.J., Maciak, R.S., Hinson, N.A., Nichols, C.L., Briggs, S.L., and Laguzza, B.C. Cancer Immunol. Immunother., **28**: 171, (1989).

The two mice expressing the highest titer antisera against the HL60/Adr cells were immunized again on day 20 with 1.2x10⁷ HL60/Adr cells. On day 24 these two mice were sacrificed and their spleens were removed and activated lymphocytes were fused to HL-1 Friendly-653 nonsecreting myeloma cells (Ventrex Laboratories, Portland, ME) according to standard hybridoma methods. *See generally* Starling, J.J., Maciak, R.S., Hinson, N.A., Nichols, C.L., Briggs, S.L., and Laguzza, B.C. Cancer Immunol. Immunother., **28**: 171, (1989). After the fusion, the cells were resuspended in 200 ml of HL-1 tissue culture medium (Ventrex) supplemented with 10% fetal calf serum, L-glutamine, 50 µg/ml gentamicin, 1x10⁻⁵ M hypoxanthine, 1x10⁻⁶ M aminopterin, and 3x10⁻⁶ M thymidine.

### C. Hybridoma Screening.

On days 11 and 12 after the fusion, the supernatants from actively growing hybridomas were screened for antibody production to the HL60/Adr cell line in a solid-phase RIA performed in substantial accordance with the method of Starling, J.J., Maciak, R.S., Hinson, N.A., Nichols, C.L., Briggs, S.L., and Laguzza, B.C., *supra.* The supernatants were also screened against HL60/Vinc and HL60/s cells. The desired MoAb was reactive against HL60/Adr cells and negative against the HL60/Vinc and HL60/s cell lines. Only 1 hybridoma out of the approximately 1,000 wells initially seeded displayed the desirable binding characteristics. This hybridoma was named. L1-Dox was determined by a commercially available isotyping kit to be an IgG3 subisotype. L1-Dox was twice cloned using the fluorescence activated cell sorting technique reported by Marder P., Maciak, R.S., Fouts, R.L., Baker, R.S., and Starling, J.J. Cytometry, **11**: 498-505, (1990).

### Characterization of the Antigen Recognized by L1-Dox

The subcellular location of the L1-Dox antigen was determined by immunofluorescence on intact and permeabilized HL60/Adr, HL60/Vinc, and HL60/s cells. HL60/Adr cells are resistant to adriamycin. HL60/Vinc cells are resistant to vinca alkaloids such as vincristine and vinblastine. The vinca-resistant phenotype is linked to mdr expression and thus is readily distinguishable from the P190 based anthracycline resistant phenotype. HL60/s cells are the parental cell line from which the drug resistant cell-lines HL60/Adr and HL60/Vinc were derived by slowly escalating the concentration of adriamycin or vinca, respectively, in the cell culture. Intact cells were obtained from the suspension culture by centrifuging the cells and washing the pellets 1X in HL-1 media containing 1% gamma globulin free horse serum (AHS). Cells were permeabilized by washing the cell pellets in PBS containing 10 mM EDTA as described by Hallden, G., Andersson, U., Hed, J., and Johansson, S.G.O. J. Immunol. Meth., **124**: 103, (1989).. The cells were resuspended in PBS containing 4% paraformaldehyde and incubated for 10 minutes at room temperature. The pellets were washed 2x in PBS + 10 mM EDTA. The pellets were resuspended in PBS containing 0.6% n-octyl-β-D-glucopyranoside, (Sigma Chemical Company, St. Louis, MO) and incubated at room temperature for 5 minutes at room temperature. The L1-Dox MoAb was added to the cell pellets and incubated for 30 minutes at 4°C. The cells were washed and the pellets were resuspended in a 1:100 dilution of rabbit anti-mouse fluorescein isothiocyanate for 30 minutes at 4°C. The cells were washed and examined under fluorescence microscopy. Intact or permeabilized HL60/Vinc or HL60/s cells did not react with the L1-Dox antibody. Intact HL60/Adr cells were also negative with the L1-Dox MoAb. Permeabilized HL60/Adr cells, however, exhibited a strong fluorescence signal which was localized in the perinuclear area in the cytoplasm. This fluorescent pattern indicated that the L1-Dox MoAb bound to an intracellular antigen which may be localized primarily in the endoplasmic reticulum. This fluorescent pattern is consistent with the previous studies of Center *et al.* Marquardt, D. and Center, M.S. Cancer Res., **52:** 3157, (1992) who demonstrated that the P190 protein associated with Adriamycin resistance was found primarily in the endoplasmic reticulum.

The molecular weight of the antigen recognized by L1-Dox was determined by autoradiography of ³⁵S-methionine labeled whole cell lysates immunoprecipitated by L1-Dox and separated into discrete bands by sodium dodecyl sulfate polyacrylamide gel electrophoresis (SDS-PAGE). HL60/s, HL60/Vinc, or HL60/Adr cells were grown in suspension culture and centrifuged. Cell pellets containing 1x10⁷ cells were resuspended in 10 ml Minimum Eagle's Medium (MEM) which was methionine deficient and was supplemented with 5% dialyzed fetal calf serum and 50 µg/ml gentamicin. The cells were centrifuged and the pellets were resuspended in 1 ml methionine deficient MEM containing 1 mCi ³⁵S, L-methionine and incubated at 37°C for 2.5 hours. The cells were centrifuged and the pellets were washed 1x in PBS. The pellets were resuspended in 1 ml cold RIPA buffer (150 mM NaCl, 1% Nonidet P-40 (Sigma 1992 Catalog Number N8265), 0.5% sodium deoxycholate, 0.1% SDS, 2% bovine serum albumin (BSA), 50 mM Tris, pH 7.5,) and incubated on ice for 30 minutes with occassional shaking. The lysates were centrifuged in an Eppendorf® microfuge for 10 minutes. The supernatants were carefully removed and frozen at -70°C. The radiolabeled lysates were immunoprecipitated using 100 µL of L1-Dox (@15 µg/mL) or 100 µL of a 100 µg/mL solution of an irrelevant IgG₃ MoAb. The MoAbs were added to 0.3 mL of radiolabled lysate (@1.2x10⁸ cpm) and incubated on ice for 30 minutes. Twenty µL of a 1:40 dilution of rabbit anti-mouse IgG, Fc-specific antibody were added and incubated for an additional 30 minutes on ice. 100 µL of a Protein A-Sepharose suspension (10% w/v in RIPA buffer) were added and the incubation was continued for an additional 60 minutes at 4°C. The samples were centrifuged for 15 seconds in the microfuge and the supernatants were discarded. The pellets were washed 2x in RIPA lysis buffer. Immune precipitated complexes were removed from the Protein A-Sepharose beads by heating the pellets for 5 minutes at 100°C in 100 µL Laemmli dialysis buffer, Laemmli, U.K. Nature **227**: 680 (1970). The samples were centrifuged for 2 minutes in the microfuge and the supernatants were collected and frozen at -70°C. The immunoprecipitated samples (5900 cpm each) were loaded onto a 4% stacking and 7.5% separating discontinuous Laemmli SDS-PAGE gel (Id.) and electrophoresced at 50 mA. At the conclusion of the SDS-PAGE the gel was placed in Coomassie brilliant blue stain overnight. The gel was destained and treated with EN³HANCE® according to the manufacturer's (Kodak) instructions. The gel was dried down and placed into a cassette with Kodak AR® X-Ray film. The film was developed and it was determined that L1-Dox immunoprecipitated a distinct band of ∼ 190 kilodaltons Mr from the HL60/Adr cell line. L1-Dox did not immunoprecipitate any bands from the HL60/s or HL60/Vinc cell lines which were distinct from the irrelevant IgG₃ subisotype matched control MoAb. These data, in conjunction with the immunofluorescence studies described above, established that L1-Dox bound to the P190 protein associated with Adriamycin resistance in the HL60/Adr cell line.

### L1-Dox Immunoreactivity

The binding specificity of L1-Dox was determined using a solid-phase RIA against a number of human tumor cell lines and immunohistochemical analysis against frozen sections of normal and tumor human tissues. L1-Dox activity was assayed against a panel of 10 human tumor cell lines which included (numbers in parentheses indicate quantity of different lines used) non small cell lung adenocarcinoma (1), a drug resistant variant of the non small cell lung adenocarcinoma cell line which expresses P-glycoprotein (1), colon carcinoma (4), small cell lung carcinoma (1), melanoma (1), squamous cell carcinoma (1), and breast carcinoma (1). L1-Dox was not reactive with any of these human tumor cell lines. L1-Dox, however, did bind to a human breast carcinoma cell line, MDA-MB-231, which was derived from a patient who had been treated with Adriamycin. The results of the studies described above are summarized in Figure 1.

Immunohistochemical analysis of L1-Dox against normal human tissues revealed that this antibody was negative against frozen tissue sections derived from colon, stomach, heart, kidney, lung, salivary gland, and pancreas. This reactivity pattern further distinguishes P190 from P-gp since P-gp is highly expressed in normal colon Pastan, I., and Gottesman, M. M. Annu. Rev. Med., **42**: 277, (1991). L1-Dox demonstrated some normal tissue reactivity with hepatocytes, tonsil epithelium, and high endothelial venules of lymph node and tonsil. L1-Dox has been tested against a panel of human tumor specimens comprised of 4 colon carcinoma and 4 breast carcinoma specimens. The MoAb was reactive with 2/4 colon carcinomas although the antibody stained less than 50% of the cells in both positive cases and in one of the two positive cases the staining was weak. The majority of the tumor cells, however, stained in all 4 breast carcinoma cases and the intensity of the staining was strong.

### Example 2

### Purification of L1-Dox

L1-Dox, an IgG3 isotype, binds to Protein A. A number of Protein A affinity matrices are commercially available from Sigma Chemical Company. CNBr Sepharose-Protein A (Sigma catalog No P3391) is used to purify L1-Dox. L1-Dox is diluted and applied to the Protein A column in Phosphate buffered saline, pH 7.0-7.2 (PBS) and the column is then washed with 3 bed volumes of PBS prior to elution with 0.2 M glycine, pH 3.0. L1-Dox elution is monitored at 280 nm and L1-Dox fractions are neutralized by addition of 1 M Tris, pH 7.0 and pooled.

### Example 3

### Preparation of an L1-Dox Immunoadsorbent

CNBr-activated Sepharose 4B is commercially available from Pharmacia Fine Chemicals AB, Box 175, S-75104 Uppsala 1, Sweden. Two grams of CNBr-Sepharose is washed and swollen using 500 ml of 1 mM HCl. The wash is performed using a scintered glass filter(G3). L1-Dox is prepared by dilution or dialysis to a concentration of 10 mg/ml in O.1 M NaHCO₃. Two milliliters of the L1-Dox in NaHCO₃ are mixed with 7 milliliters of the washed and swollen CNBr Separose, preferably in an end-over-end mixer, for 2 hours at room temperature. The L1-Dox immunoadsorbent is then gently centrifuged and the supernatant is aspirated. The L1-Dox immunoadsorbent is blocked by adding 5 ml of o.2 M glycine, pH 8. The L1-Dox immunoadsorbent is washed with alternating cycles of 10 ml of 0.1 mM NaHCO₃ and 10 ml of 0.1 M acetate buffer, pH 4 containing 0.5 M NaCl to remove excess adsorbed L1-Dox.

P190 is purified on the L1-Dox immunoadsorbent as follows. Samples containing P190 are readily determined by reactivity with L1-Dox. Samples containing P190 are solubilized and applied to the L1-Dox immunoadsorbent in PBS. The column is then washed with PBS. P190 is eluted from the column with 0.2 M glycine buffer, pH 3.0.

## Claims

1. A monoclonal antibody reactive with P190, a protein of 190,000 molecular weight, which is associated with the anthracycline and epidophyllotoxin resistant phenotype.

2. The monoclonal antibody of Claim 1, that is L1- Dox which is produced by the hybridoma deposited in the ATCC as HB11174.

3. A method for preparing P190 specific monoclonal antibodies comprising the steps of:
(a) immunizing an immunocompetent mammal with P190;
(b) fusing the lymphocytes of the immunized immunocompetent mammal with appropriate fusion partners;
(c) screening the hybridomas of step (b) for P190 reactivity; and
(d) cloning the P190 specific hybridomas of step (c).

4. A method for determining the ability of an agent to bind to P190 comprising the steps of:
(a) contacting P190 with an agent;
(b) washing the P190 of step (a) to remove unbound agent; and
(c) determining the presence or absence of the agent.

5. The method of claim 4 wherein the presence or absence of an agent is determined by the ability of the agent to prevent the binding of one or more of a panel of P190 specific antibodies.

6. A method for determining anthracycline and epipodophyllotoxin resistance comprising the steps of:
(a) contacting a tissue sample with a P190 reactive monoclonal antibody, and
(b) determining the reactivity of the P190 reactive monoclonal antibody with the tissue sample.

7. A genetically engineered antibody reactive with P190.

8. The antibody of Claim 7 that is a chimeric antibody.

9. The genetically engineered antibody of Claim 7 that is a CDR grafted antibody.

10. A method for treating epipodophyllotoxin resistant neoplasms comprising the administration of an effective amount of an agent which inhibits P190 in conjunction or in combination with an effective amount of an epipodophyllotoxin.
